# EUROPÄISCHE PATENTSCHRIFT

(11) **EP 1 275 649 B1**
(45) Veröffentlichungstag und Bekanntmachung des Hinweises auf die Patenterteilung: **08.03.2006**
(21) Anmeldenummer: 02014063.8
(22) Anmeldetag: 01.07.2002
(51) Int. Cl.: C07D 495/04, C08G 61/12

(54) **Benzodioxinothiophene, deren Herstellung und Verwendung**
Benzodioxinethiophene, its preparation and use
Benzodioxinéthiophène, sa préparation et utilisation

(30) Priorität: 12.07.2001 DE 10133927
(43) Veröffentlichungstag der Anmeldung: 15.01.2003
(73) Patentinhaber: H.C. Starck GmbH, 38642 Goslar (DE)
(72) Erfinder: Rauchschwalbe, Günter, Dr., 51375 Leverkusen (DE); Klausener, Alexander, Dr., 50259 Pulheim (DE); Kirchmeyer, Stephan, Dr., 51375 Leverkusen (DE); Reuter, Knud, Dr., 47800 Krefeld (DE)
(74) Vertreter: Feldhues, Michael L.F.

(56) Entgegenhaltungen:
- DE-A- 10 107 243
- WELSH, DEAN M. ET AL: "Fast electrochromic polymers based on new poly(3,4- alkylenedioxythiophene) derivatives" SYNTHETIC METALS (1999), 102(1-3), 967-968 , XP002256132
- KUMAR, ANIL ET AL: "Conducting Poly(3,4-alkylenedioxythiophene) Derivatives as Fast Electrochromics with High-Contrast Ratios" CHEMISTRY OF MATERIALS (1998), 10(3), 896-902 , XP002256133
- GROENENDAAL L ET AL: "POLY(3,4-ETHYLENEDIOXYTHIOPHENE) AND ITS DERIVATIVES: PAST, PRESENT, AND FUTURE" ADVANCED MATERIALS, VCH VERLAGSGESELLSCHAFT, WEINHEIM, DE, Bd. 12, Nr. 7, 4. April 2000 (2000-04-04), Seiten 481-494, XP000949752 ISSN: 0935-9648

## Beschreibung

Die Erfindung betrifft gegebenenfalls substituiertes Benzodioxinothiophen, im Folgenden als 3,4-(1 ',2'-Benzodioxy)-thiophen bezeichnet, ein Verfahren zu dessen Herstellung und dessen Verwendung zur Herstellung von elektrisch leitfähigen Oligo- oder Polymeren. Des Weiteren betrifft die Erfindung Oligo- oder Polymere, die diese Verbindungen als Wiederholungseinheit enthalten.

Organische leitfähige Polymere besitzen ein breites Anwendungsspektrum. Beispielhaft sei der Einsatz zur Herstellung von Kunststoffbatterien, von Dioden oder Transistoren oder von Solarzellen genannt. Als organische leitfähige Polymere finden beispielsweise Systeme auf Basis von Polyacetylen, Poly(p-phenylen), Polythiophen oder Polypyrrol Anwendung.

Es sind einige elektrisch leitfähige Oligo- bzw. Polymere bekannt, die aus Thiophenderivaten hergestellt werden. Bisher bekannte elektrisch leitfähige Oligo- bzw. Polymere, die aus Thiophenderivaten hergestellt werden, tragen aliphatische Substituenten. Ein besonderes Beispiel sind Polymere auf Basis von 2,3-Dihydro-thieno-<3,4-b>-<1,4>dioxin, das in der Literatur auch als 3,4-Ethylendioxy-thiophen bezeichnet wird und unter dem Namen BAYTRON® M bei der BAYER AG kommerziell erhältlich ist.

Um die Polymereigenschaften gezielt auf die jeweiligen Anforderungen einstellen zu können, ist es notwendig, eine Vielzahl geeigneter Monomer-Bausteine zur Verfügung zu haben. Es war daher Aufgabe der vorliegenden Erfindung, neue Thiophen-Derivate zur Verfügung zu stellen und Wege zu deren Herstellung aufzufinden.

Aus dem Dokument D1 (Welsh, Dean M. et al, "Fast electrochromic polymers based on new poly(3,4-alkylenedioxythiphene) derivatives", Synthetic Metals (1999), 102 (1-3), pages 967 - 698) sind Derivate der Poly(3,4alkylenedioxythiopene) bekannt geworden, von denen das BuDOT-Xyl dem Anmeldungsgegenstand am nächsten kommt. Das BuDOT-Xyl trägt jedoch an den für die elektronischen Eigenschaften wichtigen Sauerstoffatomen des mittleren Ringes benzylische Kohlenstoffatome. Diese verleihen dem Molekül aber andere als die mit der Einführung einer aromatischen Nachbargruppe nach der Erfindung verbundenen elektronischen und chemischen Eigenschaften. Beispielsweise fehlt es den benzylischen Gruppen an einem Elektronen-schiebenden Effekt in Bezug auf die Ringsauerstoffe.

Es konnten nun neue Thiophen-Derivate hergestellt werden. Dabei handelt es sich um gegebenenfalls substituiertes 3,4-(1',2'-Benzodioxy)-thiophen, wobei darunter auch Verbindungen verstanden werden, die einen Dazabenzo-Rest aufweisen. Die Verbindungen zeichnen sich unter anderem dadurch aus, dass sie eine große Anzahl verschiedenster Substituenten tragen können, die die elektronische Struktur gezielt beeinflussen, was sie als Monomere zur Herstellung leitfähiger Polymere besonders interessant macht.

Gegenstand der Erfindung sind Verbindungen der Formel I 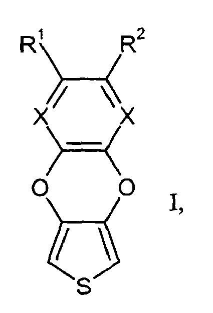
wobei
- R¹ und R²: unabhängig voneinander H, C₁-C₂₀-Alkyl, C₆-C₁₀-Aryl, C₇-C₁₂-Aralkyl, C₁-C₁₈-Alkoxy, C₂-C₁₈-Hydroxyalkylenoxy, C₂-C₃₆-Alkoxyalkylenoxy, Hydroxyethyl, Formyl, C₂-C₁₈-Acyl, Nitro, Halogen, Sulfo (-SO₃H), Cyano (-CN), Carboxy (-COOH), C₁-C₁₈-Alkoxycarbonyl oder Trifluormethyl bedeuten, oder zusammen mit dem aromatischen Ring, an den sie gebunden sind, ein anelliertes aromatisches Ringsystem ergeben und
- X: für N oder C-H steht.

Vorzugsweise bedeuten R¹ und R² unabhängig voneinander H, C₁-C₁₆-Alkyl, C₆-C₁₀₋Aryl, C₁-C₁₈-Alkoxy, C₂-C₄-Acyl, Nitro, Halogen, beispielsweise Fluor, Chlor oder Brom, oder C₂-C₃₆-Alkoxyalkylenoxy, besonders bevorzugt H, Methyl, Butyl, Phenyl, Acetyl (-CO-CH₃), Methoxy, Methoxy-ethoxy (H₃C-O-CH₂-CH₂-O-), Nitro oder Chlor. Insbesondere bevorzugt bedeutet mindestens einer der Reste R¹ und R² Wasserstoff.

Bevorzugt bedeutet X C-H.

Die Verbindungen der Formel I stellen eine wertvolle Ergänzung zu den bekannten Thiophen-Derivaten mit aliphatischen Substituenten dar, da sie sich zur Herstellung leitfähiger Polymere mit besonders günstigen Eigenschaften, insbesondere hoher Leitfähigkeit, eignen. Derartige Polymere können beispielsweise als Elektroden eingesetzt werden.

Verbindungen der Formel I, in denen X für C-H steht, können beispielsweise hergestellt werden, indem 3,4-Dialkoxythiophen, vorzugsweise das bereits bekannte 3,4-Dimethoxythiophen mit ggf. substituierten 1,2-Dihydroxybenzolen umgesetzt wird.

Diese Umsetzung kann beispielsweise bei Normaldruck in einem aromatischen Lösemittel, wie Toluol oder Xylol bei 100 bis 160°C, bevorzugt Rückflusstemperatur, in Gegenwart eines sauren Katalysators, wie z.B. Schwefelsäure oder p-Toluolsulfonsäure stattfinden.

Verbindungen der Formel I, in denen X für N steht, können beispielsweise durch Umsetzung eines bekannten 3,4-Dihydroxythiophendicarbonsäureesters mit einem ggf. substituierten 2,3-Dihalogen-1,4-diazabenzol und anschließender Verseifung und Decarboxylierung hergestellt werden.

Die Verbindungen der Formel I lassen sich zur Herstellung von elektrisch leitfähigen Polymeren verwenden. Dabei kann sowohl nur eine Verbindung der Formel I als Monomer eingesetzt werden, als auch ein Gemisch verschiedener Verbindungen, die unter die Definition der Formel I fallen. Es ist überdies möglich, neben einer oder mehrerer Verbindungen der Formel I auch weitere Thiophen-Derivate als Monomere zuzusetzen, insbesondere das bekannte 3,4-Ethylendioxy-thiophen.

Die Polymerisation erfolgt entsprechend dem Vorgehen bei der Polymerisation bekannter Thiophen-Derivate. Sie kann beispielsweise oxidativ mit Oxidationsmitteln wie Eisen-III-chlorid oder anderen Eisen-III-Salzen, H₂O₂, Natrium- oder Kaliumperoxodisulfat, Kaliumdichromat, Kaliumpermanganat oder elektrochemisch erfolgen.

Die Erfindung betrifft weiterhin die Verwendung von Verbindungen der Formel I zur Herstellung von elektrisch leitfähigen Polymeren und elektrisch leitfähige Polymere, die durch Polymerisation einer Verbindung der Formel I hergestellt werden und daher als Wiederholungseinheit wenigstens eine Verbindung der Formel I enthalten.

Die Erfindung wird im Folgenden durch Beispiele weiter erläutert, wobei die Beispiele nicht als Einschränkung des Erfindungsgedankens zu sehen sind.

### Beispiele

### Beispiel 1

45 g 3,4-Dimethoxythiophen (0,31 mol), 33 g Brenzcatechin (0,3 mol), 60 ml Xylol, 1,5 g p-Toluolsulfonsäure und 0,6 g Triphenylphosphin wurden unter Schutzgas 24 Stunden zum Sieden (145°C) erhitzt. Entstehendes Methanol wurde dabei abdestilliert.

Zur Kontrolle des Reaktionsverlaufs wurden in Abständen Proben entnommen und durch HPLC analysiert. Die Reaktion wurde abgebrochen, sobald das eingesetzte 3,4-Dimethoxythiophen vollständig abreagiert war.

Nach Abkühlen wurde viermal mit je 50 ml Cyclohexan extrahiert. Der erste Cyclohexan-Extrakt war tief dunkel rotbraun, die folgenden wurden immer heller und enthielten immer reineres Produkt. Die Extrakte wurden vereinigt, beim Stehen über Nacht fiel ein dunkel rotbraunes Öl aus, das abgetrennt wurde.

Das Öl wurde am Rotationsverdampfer eingeengt und von Xylol befreit und mit 200 ml Cyclohexan aufgenommen.

Die vereinigten Cyclohexan-Lösungen wurden mit 0,4 g Aktivkohle versetzt, auf 70°C erwärmt und abfiltriert. Die geklärte Lösung wurde am Rotationsverdampfer trocken destilliert (70°C, 25 mbar).

Es wurden 14,8 g leicht gelbstichiges Produkt mit dem Schmelzpunkt von 60°C gewonnen, das gemäß GC-MS-Untersuchung 99 % Benzo-thieno-dioxin (3,4-(1',2'-Benzodioxy)thiophen) enthielt. MS: m/e=190 (100 %).

Das Produkt wurde chromatographisch weiter gereinigt (Laufmittel: Cyclohexan, an Kieselgel als stationärer Phase). Man erhielt ein rein weißes, kristallines Produkt das bei 72°C schmilzt.

Im Infrarot-Spektrum (KBr-Pressling) absorbiert das Produkt stark bei 1509, 1493, 1281, 766 und 742 cm⁻¹.
1H-NMR (CDCl₃): 6,39 ppm (s, 2 H), 6,88 ppm (s, 4 H).

Die Verbindung kann z.B. elektrochemisch zu einem elektrisch leitenden Film polymerisiert werden oder durch Oxidation mit Fe-(III)-tosylat in Ethanol zunächst polymerisiert und dann aus Chloroform zu einem elektrisch leitenden Film verarbeitet werden.

### Beispiel 2

12,5 g 3,4-Di-n-propyloxy-thiophen, 20,7 g 4-tert.-Butylbrenzcatechin und 1,2 g p-Toluolsulfonsäure-monohydrat wurden in 90 ml Toluol unter einer Schutzgasatmosphäre 3 Stunden zum Rückfluss erhitzt. Anschließend wurde ein Gemisch aus Toluol und gebildetem n-Propanol abdestilliert. Die abdestillierte Menge Lösungsmittel wurde portionsweise zunächst durch Toluol, dann durch Xylol ersetzt. Insgesamt wurde 12 Stunden lang destilliert. Der Reaktionsverlauf wurde mittels Dünnschichtchromatografie überwacht.

Nach beendeter Reaktion wurde abgekühlt und die erhaltene dunkle Lösung dreimal mit Wasser gewaschen. Das Lösungsmittel wurde am Rotationsverdampfer im Vakuum abdestilliert und der schwarze Rückstand durch präparative Säulenchromatografie (an SiO₂ (Kieselgel) als stationäre Phase, mit Toluol als Laufmittel) gereinigt. Es wurden 16,6 g eines dunkelroten Öles erhalten, das laut 1H-NMR 65 % 3,4-(4'-tert.-Butyl-benzo- 1',2'-dioxy)-thiophen 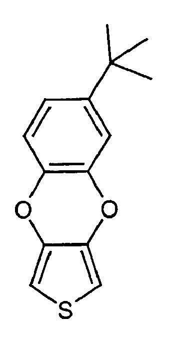
enthielt.
1H-NMR (CDCl₃): 1,28 ppm (9H, s), 6,40 ppm (2H, s), 6,8-6,95 ppm (2H, aromat. AB-System), 6,94 ppm (1H, s).

### Beispiel 3: Polymerisation von 3,4-(1',2'-Benzodioxy)thiophen

In 10,4 g Ethanol wurden 5,0 g Eisen-(III)-p-toluol-sulfonat gelöst. Zu der Lösung wurden 0,58 g 3,4-(1'2'-Benzodioxy)thiophen zugegeben. Es wurde 1 Stunde bei Raumtemperatur gerührt und die Lösung anschließend auf eine Glasplatte oder ein anderes Substrat gegossen und die Schicht beispielsweise durch spin-coating zu einem Film ausgedünnt. Anschließend wurde 1 Stunde bei 75°C getrocknet.

Man erhielt einen dunkelblauen Film mit leicht violettem Farbeinschlag. Der Film war etwas dunkler als ein Vergleichsfilm, der unter gleichen Bedingungen aus 3,4-Ethylendioxythiophen erhalten wurde.

Der Film zeigte elektrische Leitfähigkeit.

## Patentansprüche

1. Verbindungen der Formel I 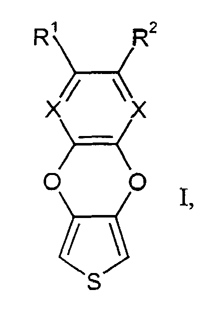
wobei
R¹ und R² unabhängig voneinander H, C₁-C₂₀-Alkyl, C₆-C₁₀-Aryl, C₇-C₁₂₋Aralkyl, C₁-C₁₈-Alkoxy, C₂-C₁₈-Hydroxyalkylenoxy, C₂-C₃₆-Alkoxyalkylenoxy, Hydroxyethyl, Formyl, C₂-C₁₈-Acyl, Nitro, Halogen, Sulfo (-SO₃H), Cyano (-CN), Carboxy (-COOH), C₁-C₁₈-Alkoxycarbonyl oder Trifluormethyl bedeuten, oder zusammen mit dem aromatischen Ring, an den sie gebunden sind, ein anelliertes aromatisches Ringsystem ergeben und
X für N oder C-H steht.

2. Verbindung gemäß Anspruch 1, **dadurch gekennzeichnet, dass** R¹ und R² unabhängig voneinander H, C₁-C₁₆-Alkyl, C₆-C₁₀-Aryl, C₁-C₁₈-Alkoxy, C₂-C₄₋Acyl, Nitro, Halogen oder C₂-C₃₆-Alkoxyalkylenoxy bedeuten.

3. Verbindung gemäß Anspruch 2, **dadurch gekennzeichnet, dass** R¹ und R² unabhängig voneinander H, Methyl, Butyl, Phenyl, Acetyl (-CO-CH₃), Methoxy, Methoxy-ethoxy, Nitro oder Chlor bedeuten.

4. Verbindung gemäß Anspruch 1, **dadurch gekennzeichnet, dass** wenigstens einer der Reste R¹ und R² H bedeutet.

5. Verbindung gemäß Anspruch 1, **dadurch gekennzeichnet, dass** X für C-H steht.

6. Verfahren zur Herstellung einer Verbindung gemäß wenigstens eines der Ansprüche 1 bis 5, **dadurch gekennzeichnet, dass** ein 3,4-Dialkoxythiophen mit einem gegebenenfalls substituierten 1,2-Dihydroxybenzol umgesetzt wird.

7. Verwendung einer Verbindung gemäß wenigstens eines der Ansprüche 1 bis 5 zur Herstellung eines elektrisch leitfähigen Polymers.

8. Elektrisch leitfähiges Polymer, **dadurch gekennzeichnet, dass** es als Wiederholungseinheit wenigstens eine Verbindung gemäß Anspruch 1 enthält.

## Claims

1. Compounds of formula I 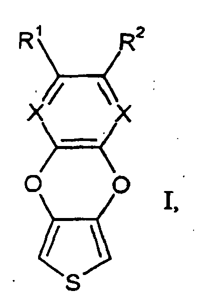
wherein
R¹ and R², independently of one another, signify H, C₁-C₂₀ alkyl, C₆-C₁₀ aryl, C₇-C₁₂ aralkyl, C₁-C₁₈ alkoxy, C₂-C₁₈ hydroxyalkyleneoxy, C₂-C₃₆ alkoxyalkyleneoxy, hydroxyethyl, formyl, C₂-C₁₈ acyl, nitro, halogen, sulfo (-SO₃H), cyano (-CN), carboxy (-COOH), C₁-C₁₈ alkoxycarbonyl or trifluoromethyl, or form an anellated aromatic ring system together with the aromatic ring to which they are bonded and
X denotes N or C-H.

2. The compound according to claim 1, **characterised in that** R¹ and R², independently of one another, signify H, C₁-C₁₆ alkyl, C₆-C₁₀ aryl, C₁-C₁₈ alkoxy, C₂-C₄ acyl, nitro, halogen or C₂-C₃₆ alkoxyalkyleneoxy.

3. The compound according to claim 2, **characterised in that** R¹ and R², independently of one another, signify H, methyl, butyl, phenyl, acetyl (-CO-CH₃), methoxy, methoxyethoxy, nitro or chlorine.

4. The compound according to claim 1, **characterised in that** at least one of the residues R¹ and R² signifies H.

5. The compound according to claim 1, **characterised in that** X denotes C-H.

6. A process for the production of a compound according to at least one of claims 1 to 5, **characterised in that** a 3,4-dialkoxythiophene is reacted with an optionally substituted 1,2-dihydroxybenzene.

7. The use of a compound according to at least one of claims 1 to 5 for the production of an electrically conductive polymer.

8. An electrically conductive polymer, **characterised in that** it contains at least one compound according to claim 1 as a repeating unit.

## Revendications

1. Composés de la formule I 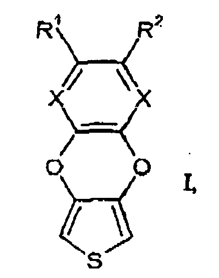
où
R¹ et R² représentent indépendamment l'un de l'autre H, un groupe alkyle en C₁-C₂₀, un groupe aryle en C₆-C₁₀, un groupe aralkyle en C₇-C₁₂, un groupe alcoxy en C₁-C₁₈, un groupe hydroxyalkylènoxy en C₂-C₁₈, un groupe alkoxyalkylènoxy en C₂-C₃₆, l'hydroxyéthyle, le formyle, un groupe acyle en C₂-C₁₈, un groupe nitro, un halogène, un groupe sulfo (-SO₃H), un groupe cyano (-CN), un groupe carboxyle (-COOH), un groupe (alkoxy en C₁-C₁₈) carbonyle ou un groupe trifluorométhyle, ou donnent ensemble avec l'anneau aromatique auquel ils sont liés une combinaison cyclique aromatique condensée et
X représente N ou C-H.

2. Composé suivant la revendication 1, **caractérisé en ce que** R¹ et R² représentent indépendamment l'un de l'autre H, un groupe alkyle en C₁-C₁₆, un groupe aryle en C₆-C₁₀, un groupe alkoxy en C₁-C₁₈, un groupe acyle en C₂-C₄, un groupe nitro, un halogène ou un groupe alkoxyalkylènoxy en C₂-C₃₆.

3. Composé suivant la revendication 1, **caractérisé en ce que** R¹ et R² représentent indépendamment l'un de l'autre H, un groupe méthyle, un groupe butyle, un groupe phényle, un groupe acétyle (-CO-CH₃), un groupe méthoxy, un groupe méthoxy-éthoxy, un groupe nitro ou le chlore.

4. Composé suivant la revendication 1, **caractérisé en ce qu'**au moins un des restes R¹ et R² représente H.

5. composé suivant la revendication 1, **caractérisé en ce que** X représente CH.

6. Procédé de fabrication d'un composé suivant au moins une des revendications 1 à 5, **caractérisé en ce qu'**un 3,4-dialkoxythiophène est mis en réaction avec un 1,2-dihydroxybenzène, le cas échéant substitué.

7. Utilisation d'un composé suivant au moins une des revendications 1 à 5 pour la fabrication d'un polymère électriquement conducteur.

8. Polymère électriquement conducteur, **caractérisé en ce qu'**il contient comme unité répétitive au moins un composé suivant la revendication 1.
